(19) Europäisches Patentamt
European Patent Office
Office européen des brevets

(11) Numéro de publication : 0 401 134 B1

# (12) FASCICULE DE BREVET EUROPEEN

(45) Date de publication du fascicule du brevet : **06.04.94 Bulletin 94/14**

(51) Int. Cl.[5] : **C07D 211/90,** C08K 5/3435

(21) Numéro de dépôt : **90420253.8**

(22) Date de dépôt : **29.05.90**

## (54) Nouveaux composés à fonction dihydropyridine.

(30) Priorité : **31.05.89 FR 8907415**

(43) Date de publication de la demande : **05.12.90 Bulletin 90/49**

(45) Mention de la délivrance du brevet : **06.04.94 Bulletin 94/14**

(84) Etats contractants désignés : **AT BE CH DE DK ES FR GB GR IT LI LU NL SE**

(56) Documents cités :
**DE-A- 2 017 825**
**US-A- 4 214 088**

(73) Titulaire : **GREAT LAKES CHEMICAL FRANCE S.A.**
**5, rue de la Grand Ourse**
**F-95800 Cergy-Saint-Christophe (FR)**

(72) Inventeur : **Gay, Michel**
**10 Rue Henri Rolland**
**F-69100 Villeurbanne (FR)**
Inventeur : **Lavault, Sylvie**
**17 rue Jassron**
**F-69003 Lyon (FR)**

(74) Mandataire : **Koch, Gustave et al**
**Cabinet PLASSERAUD 84, rue d'Amsterdam**
**F-75009 Paris (FR)**

EP 0 401 134 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux composés à fonction dihydropyridine.

Elle concerne également l'utilisation de ces composés pour la stabilisation thermique et lumière des polymères.

Le brevet FR-A-2 239 496 décrit des diméthyl-2,6 dicarboxylate-3,5 dihydro-1,4 pyridines comme stabilisants thermiques du polychlorure de vinyle (PVC).

Le brevet EP-a-0 005 678 décrit une synergie entre les diméthyl-2,6 dicarboxylate-3,5 dihydro-1,4 pyridines et les β-dicétones pour la stabilisation thermique du PVC.

La demande de brevet allemand DEA 2 017 825 décrit comme photostabilisant bis (benzoyl-4 hydroxy-3phénoxy)-2 ethoxycarbonyl)-1,3 benzène.

La demande US 4 214 088 décrit des diméthyl-2,6 dicarboxylate-3,5 dihydro-1,4 pyridines qui sont utilisées comme stabilisants thermiques.

De ces enseignements il n'était toutefois pas possible d'inférer une molécule qui ait des qualités de stabilisant à la fois contre la lumière et contre la dégradation par traitement thermique.

Ces composés de la dihydro-1,4 pyridine ont une bonne efficacité pour la stabilisation thermique du PVC. Ils sont cependant insuffisants pour certaines applications où le polymère est soumis à des expositions extérieures, c'est-à-dire lorsqu'il est nécessaire d'avoir une stabilité lumière.

La présente invention concerne de nouveaux composés à fonction dihydro-1,4 pyridine de formule générale (I) :

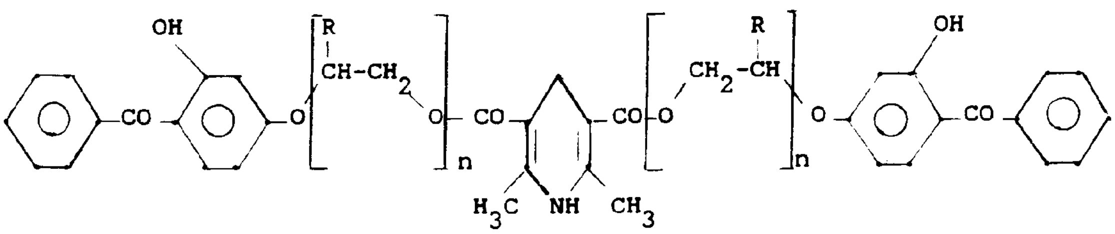

(I)

dans laquelle :

- n est un nombre de 1 à 3
- R représente un radical méthyle ou un atome d'hydrogène. La synthèse des composés de formule (I) peut être effectuée à partir d'hydroxy-2 hydroxyalcoxy-4 benzophénone (ou d'hydroxy-2 hydroxy (polyalcoxy)-4 benzophénone et de dicétène pour former l'acétoacétate correspondant de la benzophénone substituée.

On fait ensuite réagir l'acétoacétate ainsi obtenu avec de l'acétate d'ammonium en présence d'hexaméthylène tétramine selon le porcédé connu permettant de préparer les diméthyl-2,6 dicarboxylate-3,5 dihydro-1,4 pyridines à partir des acétoacétates correspondants.

D'une façon plus générale, les composés de formule (I) sont obtenus selon la méthode de HANTZCH en deux étapes :

- réaction du formaldéhyde sur l'acétoacétate conduisant au méthylène bis-acétoacétate de la benzophénone substituée ;
- cyclisation de ce dernier composé en dihydro-1,4 pyridine correspondante au moyen d'ammoniac.

On peut aussi, en inversant ces deux étapes, faire réagir le formaldéhyde sur le β-aminocrotonate dérivant de l'acétoacétate.

Parmi les composés de formule (I) les composés les plus facilement accessibles sont ceux pour lesquels n = 1, c'est-à-dire :

- la bis [(benzoyl-4 hydroxy-3 phénoxy)-2 éthoxycarbonyl]-3,5 diméthyl-2,6 dihydro-1,4 pyridine ;
- la bis [(benzoyl-4 hydroxy-3 phénoxy)-2 méthyl-2 éthoxycarbonyl]-3,5 diméthyl-2,6 dihydro-1,4 pyridine.

Les composés de formule (I) peuvent être utilisés comme stabilisants thermiques et lumière dans les polymères organiques.

Ainsi on peut les utiliser dans les polymères halogénés et notamment dans les polymères chlorés. Ils ont une action de stabilisation thermique de ces polymères et une action de protection contre la lumière et en particulier contre les radiations ultra-violettes (UV). Ils peuvent être utilisés seuls ou associés à l'autres stabilisants thermiques comme par exemple les dérivés organiques de l'étain.

Les composés de l'invention sont également souvent associés dans les polymères chlorés à d'autres stabilisants thermiques primaires.

De préférence, ces stabilisants primaires sont des dérivés organiques du zinc, du calcium, du baryum, du magnésium et du strontium et le cas échéant les hydrotalcites.

Un des objets de l'invention consiste donc en des compositions à base de polymère chloré stabilisé, caractérisées en ce qu'elles contiennent :

a) une quantité efficace d'au moins un composé organique du zinc,

b) une quantité efficace d'au moins un composé organique du calcium, du baryum, du magnésium ou du strontium et/ou d'une hydrotalcite ;

c) une quantité efficace d'au moins un composé à fonction dihydro-1,4 pyridine de formule (I).

Les polymères chlorés sont notamment le polychlorure de vinyle (PVC), le polychlorure de vinylidène ; les copolymères comportant majoritairement des motifs chlorure de vinyle obtenus à partir de chlorure de vinyle et d'autres monomères ; les mélanges de polymères ou copolymères dont une partie majoritaire est obtenue à partir de chlorure de vinyle.

De manière générale tout type de PVC convient, quel que soit son mode de préparation : polymérisation en masse, en suspension, en dispersion ou de tout autre type et quelle que soit sa viscosité intrinsèque.

Les homopolymères du chlorure de vinyle peuvent également être modifiés chimiquement, par exemple par chloration.

De nombreux copolymères du chlorure de vinyle peuvent également être stabilisés contre les effets de la chaleur, c'est-à-dire le jaunissement et la dégradation. Ce sont en particulier les copolymères obtenus par copolymérisation du chlorure de vinyle avec d'autres monomères présentant une liaison éthylénique polymérisable, comme par exemple l'acétate de vinyle ou le chlorure de vinylidène ; les acides maléique ou fumarique ou leurs esters ; les oléfines telles que l'éthylène, le propylène, l'hexène ; les esters acryliques ou méthacryliques ; le styrène ; les éthers vinyliques tels que le vinyldodécyléther.

Habituellement ces copolymères contiennent au moins 50 % en poids de motifs chlorure de vinyle et de préférence au moins 80 % en poids de motifs chlorure de vinyle.

Les compositions selon l'invention peuvent également contenir des mélanges à base de polymère chloré contenant des quantités minoritaires d'autres polymères, comme les polyoléfines halogénés ou les copolymères acrylonitrile-butadiène-styrène.

Le PVC seul ou en mélange avec d'autres polymères est le polymère chloré le plus largement utilisé dans les compositions de l'invention.

Les composés organiques du zinc sont de préférence les carboxylates et les phénolates de zinc.

Les plus couramment utilisés sont par exemple les sels de zinc des acides maléique, acétique, diacétique, propionique, hexanoïque, éthyl-2 hexanoïque, décanoïque, undécanoïque, laurique, myristique, palmitique, stéarique, oléïque, ricinoléïque, béhénique, hydroxystéarique, hydroxyundécanoïque, benzoïque, phénylacétique, paratertiobutylbenzoïque et salicylique; les phénolates de zinc du phénol et des phénols substitués par un ou plusieurs radicaux alkyle, tels que les nonylphénols.

Pour des raisons pratiques ou pour des raisons économiques, on choisit de préférence parmi les composés organiques du zinc cités précédemment, le propionate de zinc, l'éthyl-2 hexanoate de zinc, le laurate de zinc, le stéarate de zinc, l'oléate de zinc, le ricinoléate de zinc, le benzoate de zinc, le paratertiobutylbenzoate de zinc, le salicylate de zinc, le maléate de zinc et de mono(éthyl-2 hexyle), les nonylphénates de zinc.

Généralement les composés organiques du zinc représentent de 0,005 % à 1 % en poids par rapport au polymère chloré, et de préférence de 0,01 % à 0,6 % en poids.

Les composés organiques du calcium, du baryum, du magnésium et du strontium sont de préférence les carboxylates et les phénolates de ces métaux.

Les plus couramment utilisés sont par exemple les sels de calcium, baryum, magnésium et strontium des acides maléique, acétique, diacétique, propionique, hexanoïque, éthyl-2 hexanoïque, décanoïque, undécanoïque, laurique, myristique, palmitique, stéarique, oléïque, ricinoléique, béhénique, hydroxystéarique, hydroxyundécanoïque, benzoïque, phénylacétique, paratertiobutylbenzoïque et salicylique ; les phénolates de calcium, baryum, magnésium et strontium du phénol et des phénols substitués par un ou plusieurs radicaux alkyle, tels que les nonylphénols.

Pour des raisons pratiques ou pour des raisons économiques, on choisit de préférence parmi les composés organiques de calcium, de baryum, de magnésium et de strontium cités précédemment les sels de calcium, de baryum et de magnésium des acides propionique, éthyl-2 hexanoïque, laurique, stéarique, oléïque, ricinoléïque, benzoïque, paratertiobutylbenzoïque, salicylique, du maléate de mono(éthyl-2 hexyle) ainsi que les nonylphénates de calcium, baryum et magnésium.

Généralement les composés organiques du calcium, du baryum, du magnésium et du strontium ou les hydrotalcites représentent de 0,005 % à 5 % en poids par rapport au polymère chloré et de préférence de 0,02 % à 2 % en poids.

Pour les applications alimentaires et notamment pour les bouteilles en PVC, on utilisera les composés organiques du calcium ou les mélanges composés organiques du calcium/composés organiques du magnésium.

Les hydrotalcites que l'on peut introduire dans les compositions à base de polymère chloré selon l'invention, à la place des composés organiques de calcium, de baryum, de magnésium et de strontium ou conjointement à ces composés, sont notamment les composés qui ont été décrits dans le brevet français FR-A-2 483 934 et dans le brevet européen EP-A-0 063 180.

Généralement les compositions à base de polymère chloré comprennent de 0,005 % à 5 % en poids de composé de formule (I) par rapport au polymère chloré.

De préférence elles contiennent de 0,01 % à 2 % en poids de composé de formule (I) par rapport au polymère chloré.

Par rapport aux dihydro-1,4 pyridines de l'art antérieur utlisées comme stabilisants thermiques des polymères chlorés, les composés de formule (I) présentent sensiblement la même efficacité au plan de la stabilisation thermique pour un même poids, c'est-à-dire pour une plus faible quantité de motifs dihydro-1,4 pyridine, tout en ayant en outre une action efficace de protection contre les rayonnements UV.

Dans les compositions à base de polymère chloré de l'invention, il est naturellement possible d'introduire également d'autres additifs.

C'est ainsi notamment le cas des β-dicétones ou β-dicétoaldéhydes qui ont une action synergique avec les composés à fonction dihydropyridine.

Ces β-dicétones ont été notamment décrites dans les brevets et certificats d'addition français publiés sous les numéros FR 2 292 227, FR 2 324 681, FR 2 351 149, FR 2 352 025, FR 2 383 988 et FR 2 456 132 et dans les brevets européens EP 0 040 286 et EP 0 046 161.

Comme exemples non limitatifs de telles β-dicétones, on peut citer le benzoylstéaroylméthane, le dibenzoylméthane, la benzoylacétone, le benzoyl méthyl-3 butanoylméthane, les méthoxycarbonylbenzoylbenzoylméthanes, les bis-β-dicétones telles que le bis(acétylacéto)-1,4 butane, le bis(benzoylacéto)-1,8 octane, le bis(acétylacéto)-1,4 benzène.

Lorsqu'elles sont présentes, les β-dicétones représentent de 0,005 % à 5 % en poids par rapport au polymère chloré et de préférence de 0,01 % à 2 % en poids.

Les compositions de l'invention peuvent comporter d'autres stabilisants thermiques secondaires tels que les polyols, les phosphites, les composés époxydés.

Les polyols ont généralement l'avantage d'allonger la durée de vie des polymères chlorés soumis à un traitement thermique.

Généralement il est préférable que les polyols utilisés aient un point d'ébullition supérieur à 150°C et de préférence supérieur à 170°C, à cause de la mise en oeuvre à température élevée des polymères chlorés.

A titre d'exemples de tels polyols on peut citer des triols comme le triméthylolpropane, le glycérol, l'hexanetriol-1,2,6, le butanetriol-1,2,4, le trishydroxyéthylisocyanurate ; des tétrols comme le pentaérythritol, le diglycérol ; des pentitols comme le xylitol, le tétraméthylolcyclohexanol ; des hexitols comme le mannitol, le sorbitol, le dipentaérythritol ; des polyols partiellement estérifiés par un acide carboxylique et dans la formule desquels au moins 3 fonctions hydroxyle sont libres ; des alcools polyvinyliques, notamment ceux dans lesquels il reste moins de 30 % en moles de groupes esters par rapport à l'ensemble de leurs groupes esters et hydroxyle et qui présentent une viscosité à 20°C en solution aqueuse à 4 % en poids comprise entre environ 4 x $10^{-3}$Pa.s et 60 x $10^{-3}$Pa.s.

Parmi ces polyols les préférés sont le xylitol, le mannitol, le sorbitol, le tétraméthylolcyclohexanol et les alcools polyvinyliques définis précédemment.

Lorsqu'il est présent dans les compositions selon l'invention, on utilise en général de 0,005 % à 1 % en poids de polyol par rapport au polymère chloré et de préférence de 0,01 % à 0,6 % en poids.

Les époxydes qui peuvent être utilisés dans les compositions selon l'invention sont généralement des composés complexes, habituellement des polyglycérides époxydés comme l'huile de soja époxydée qui est la plus fréquemment employée, l'huile de lin époxydée, les huiles de poisson époxydées, la talloil époxydée.

Les compositions selon l'invention peuvent comporter également des phosphites organiques, notamment des phosphites aliphatiques ou des phosphites aromatiques ou des phosphites mixtes aliphatiques et aromatiques.

Parmi les plus intéressants, on peut citer :

- les diphosphites de pentaérythrityle et de dialkyle,
- les diphosphites de pentaérythrityle et de diphényle,
- les diphosphites de pentaérythrityle et de bis(ditertiobutyl-2,4 phényle),
- les diphosphites de tétraalkyle et de bis(phénylène-1,4) diméthylméthane,
- les diphosphites de tétraalkyle et de bis(dialkyl-2,5 phénylène-1,4) alkylméthane,
- le diphosphite de diphényle, bis{(butoxy-2 éthoxy)-2 éthyle} et 4,4'-isopropylidènediphényle ;

- le diphosphite de tétrakis{(butoxy-2 éthoxy)-2 éthyle} et 4,4′-isopropylidènediphényle ;
- le triphosphite de diphényle, tris{(butoxy-2 éthoxy)-2 éthyle} et bis(4,4′-isopropylidènediphényle) ;
- le tétraphosphite de diphényle, tétrakis{(butoxy-2 éthoxy)-2 éthyle} et tris(4,4′-isopropylidènediphényle) ;
- le diphosphite de diphényle, bis{(butoxy-2 éthoxy)-2 éthyle} et 4,4′-isopropylidènediphényle ;
- le diphosphite de tétrakis{(butoxy-2 éthoxy)-2 éthyle} et 4,4′-isopropylidènediphényle ;
- le triphosphite de diphényle, tris{(butoxy-2 éthoxy)-2 éthyle} et bis(4,4′-isopropylidènediphényle) ;
- le tétraphosphite de diphényle, tétrakis{(butoxy-2 éthoxy)-2 éthyle} et tris(4,4′-isopropylidènediphényle) ;
- le pentaphosphite de diphényle, pentakis{(butoxy-2 éthoxy)-2 éthyle} et tétrakis(4,4′-isopropylidènediphényle) ;
- l'hexaphosphite de diphényle, hexakis{(butoxy-2 éthoxy)-2 éthyle} et pentakis(4,4′-isopropylidènediphényle) ;
- le triphosphite de pentakis{(butoxy-2 éthoxy)-2 éthyle} et bis(4,4′-isopropylidènediphényle) ;
- le tétraphosphite d'hexakis{(butoxy-2 éthoxy)-2 éthyle} et tris(4,4′-isopropylidènediphényle) ;
- le diphosphite de bis(ditertiobutyl-2,4 phényle), bis{(butoxy-2 éthoxy)-2 éthyle} et 4,4′-isopropylidènediphényle) ;
- le diphosphite de bis(ditertiobutyl-2,6 phényle), bis{(butoxy-2 ethoxy)-2 éthyle} et 4,4′-isopropylidènediphényle).

Lorsqu'il est présent, le phosphite représente généralement de 0,05 % à 5 % en poids par rapport au polymère chloré et de préférence de 0,1 % à 2 % en poids.

Les compositions selon l'invention peuvent également comporter des adjuvants habituels tels que des antioxydants phénoliques ; des agents anti-UV tels que les benzophénones, les benzotriazoles ou les amines stériquement encombrées (habituellement connues sous le terme de HALS).

Les compositions de l'invention peuvent être des formulations rigides, c'est-à-dire sans plastifiant ou semirigides, c'est-à-dire avec des teneurs en plastifiant réduites, telles que pour les applications dans le bâtiment ou pour la fabrication de bouteilles. Mais les compositions selon l'invention peuvent également être utilisées dans des formulations plastifiées telles que pour la fabrication de films à usage agricole.

De manière habituelle, l'incorporation des différents stabilisants ou adjuvants est faite sur le polymère chloré à l'état de poudre.

On peut bien entendu préparer un mélange de 2 ou plusieurs des composés constitutifs des compositions selon l'invention avant leur incorporation dans le polymère chloré.

Toutes les méthodes usuelles d'incorporation des différents stabilisants ou adjuvants dans le polymère peuvent être utilisées. Par exemple l'homogénéisation de la composition polymérique peut être réalisée sur malaxeur ou mélangeur à rouleaux, à une température telle que la composition devienne fluide, normalement entre 150°C et 200°C pour le PVC et pendant une durée suffisante, de l'ordre de quelques minutes à quelques dizaines de minutes.

Les compositions de polymère chloré, et plus particulièrement de PVC, peuvent être mises en oeuvre selon toutes les techniques utilisées habituellement comme par exemple l'extrusion, l'injection, l'extrusion-soufflage, le calandrage ou le moulage par rotation.

Les composés de formule (I) peuvent également être utilisés dans d'autres polymères organiques.

Ainsi ils peuvent être utilisés comme anti-UV dans les polyoléfines, les polystyrènes, les polyalcadiènes, les polyuréthannes, les polyamides, les polyesters, les polycarbonates, les polysulfones, les polyéthers-sulfones, les polyéthers-cétones, les polymères acryliques, leurs copolymères et leurs mélanges.

Les composés de formule (I) sont plus particulièrement utilisés dans les polyoléfines et les polyalcadiènes tels que le polypropylène, le polyéthylène haute densité, le polyéthylène basse densité, le polyéthylène basse densité linéaire, le polybutadiène, leurs copolymères ou leurs mélanges.

Généralement, on met en oeuvre de 0,005 % à 5 % en poids de composé de formule (I) par rapport au poids de polymère organique et de préférence de 0,01 % à 2 % en poids.

Ces compositions de polymères organiques contenant les composés de formule (I) peuvent contenir en outre les additifs et stabilisants habituellement utilisés tels que les antioxydants, les stabilisants à la lumière, d'autres absorbeurs de rayons UV, les désactivants de métaux, les phosphites et phosphonites organiques, les destructeurs de peroxydes, les charges, les plastifiants, les lubrifiants, les émulsionnants, les pigments, les azurants optiques, les ignifugeants, les antistatiques et les porogènes.

Les exemples qui suivent illustrent l'invention.

EXEMPLE 1

1a - Synthèse de l'acétoacétate de l'hydroxyéthoxy-4 hydroxy-2 benzophénone

Dans un ballon tricol de 2000 $cm^3$ muni d'un agitateur central, d'un réfrigérant, d'une ampoule de coulée et d'une tubulure latérale d'arrivée d'azote on charge 258 g (1 mole) d'hydroxyéthoxy-4 hydroxy-2 benzophénone et 1250 $cm^3$ de toluène sec.

On établi un balayage à l'azote de façon à placer la masse réactionnelle sous atmosphère inerte. On chauffe à 50°C : à cette température la masse réactionnelle est homogène.

On coule alors en 1 heure, 88 g de dicétène (1,05 mole) sous agitation, en maintenant la température entre 55 et 60°C. Après la fin de coulée on maintient la température à 60°C pendant encore 3 heures.

On élimine ensuite le toluène sous pression réduite en terminant à 70°C sous 130 à 260 Pa. Au refroidissement l'acétoacétate formé se prend en masse ; on obtient ainsi 341,7 g d'un produit brut orangé de PF : 70°C. La structure est confirmée par RMN du proton et par spectre IR ; la pureté est supérieure à 95 % ; (Rendement chimique > 95 %).

1b - Synthèse de la bis[(benzoyl-4 hydroxy-3 phénoxy)-2 éthoxycarbonyl]-3,5 diméthyl-2,6 dihydro-1,4 pyridine

Dans un ballon tricol de 4 litres équipé d'un agitateur central, d'un réfrigérant ascendant et d'une tubulure latérale d'arrivée d'azote on charge 336 g du produit obtenu à l'exemple précédent (soit 0,982 mole), 1500 $cm^3$ d'un mélange isopropanol/$H_2O$ à 20 % d'eau en volume ; on chauffe à 70 - 75°C de façon à obtenir une solution homogène ; on charge alors 33,3 g d'acétate d'ammonium et 13,75 g d'hexaméthylènetétramine.

On chauffe à reflux pendant 2 heures ; il se forme rapidement un précipité jaune au cours de cette opération. On laisse refroidir, on filtre le pricipité et on le lave 3 fois par 200 $cm^3$ de mélange isopropanol/$H_2O$ 80/20 ; on sèche en étuve sous vide à 50°C et on obtient ainsi 317,6 g (rendement 95,5 %) du produit attendu sous forme d'un solide jaune de point de fusion 188°C.

Son spectre d'absorption UV présente les caractéristiques suivantes :

- $\lambda$ maximum : 238 nm ($\varepsilon$ = 30750 $l.mol^{-1}.cm^{-1}$)
  287 nm ($\varepsilon$ = 30900 $l.mol^{-1}.cm^{-1}$)
  330 nm ($\varepsilon$ = 22500 $l.mol^{-1}.cm^{-1}$)

EXEMPLES 2 à 4 et essais comparatifs - Stabilisation thermique du PVC

On prépare la composition de base A suivante :

- PVC en poudre préparé par polymérisation ou suspension et commercialisé sous la marque LACQVYL SO 71 S (indice de viscosité selon la norme NF T 51013:80) : 1.000 g
- renforçateur de choc (copolymère butadiène/styrène/méthacrylate de méthyle) : 80 g
- lubrifiant à base d'ester de colophane (cire E) : 10 g
- huile de soja époxydée : 40 g
- stéarate de calcium : 2,5 g
- stéarate de zinc : 2,5 g

Après homogénéisation en mélangeur rapide à froid, on prélève 5 fractions de cette composition A. A chaque fraction on ajoute une quantité de composé préparé dans l'exemple 1b (DHP ex 1b) ou de diméthyl-2,6 bis(dodécyloxycarbonyl)-3,5 dihydro-1,4 pyridine (DHP art antérieur) (essais comparatifs). Les quantités en poids pour 100 g de PVC sont indiquées dans le tableau I ci-après.

On prépare à l'aide des différentes compositions ainsi obtenues, ainsi qu'avec la composition A non modifiée, des feuilles de 1 mm d'épaisseur par malaxage sur un mélangeur à 2 cylindres pendant 3 min à 180°C.

A partir d'éprouvettes (environ 1 cm x 2 cm) découpées dans ces feuilles, on effectue un test de vieillissement thermique en étuve ventilée à 180°C et l'on suit en fonction du temps l'évolution de la coloration GARDNER;

Le tableau I rassemble les indices de coloration GARDNER mesurés pour différentes durées de vieillissement ainsi que la durée avant noircissement total des échantillons testés.

L'examen des résultats montre qu'à quantité pondérale égale (donc à quantité de fonctions dihydro-1,4 pyridine beaucoup plus faible) le composé de l'invention (préparé dans l'exemple 1b) a une efficacité égale à celle d'une dihydropyridine de l'art antérieur utilisée habituellement.

| ESSAIS | STABILISANTS | | INDICES GARDNER EN FONCTION DU TEMPS EN MIN | | | | | | | |
|---|---|---|---|---|---|---|---|---|---|---|
| | Nature | Poids en g/100g PVC | 0 | 7 | 14 | 21 | 30 | 45 | 60 | Noir à |
| Témoin | Néant | -- | 2 | 6 | 7 | 7 | 7 | 8 | 10 | 69 min |
| Exemple 2 | DHP ex 1b | 0,10 | 1,5 | 2,5 | 2,5 | 2,5 | 3 | 5 | 9 | 69 min |
| Exemple 3 | DHP ex 1b | 0,20 | 1 | 2 | 2 | 2,5 | 3 | 5 | 9 | 69 min |
| Exemple 4 | DHP ex 1b | 0,40 | 0,5 | 1,5 | 1,5 | 2 | 2 | 3 | 7 | 67 min |
| Essai A1 | DHP art antérieur | 0,20 | 1 | 2 | 2 | 2,5 | 3 | 5 | 9 | 69 min |
| Essai A2 | DHP art antérieur | 0,40 | 0,5 | 1,5 | 1,5 | 2 | 2 | 3 | 7 | 67 min |

TABLEAU I

Exemples 5 et 6 et essais comparatifs - Stabilisation thermique du PVC

On prépare la composition de base B suivante :

- PVC en poudre préparé par polymérisation ou suspension et commercialisé sous la marque LACQVYL SO 71 S (indice de viscosité selon la norme NF T 51013:80) : 1.000 g

- renforçateur de choc (copolymère butadiène/styrène/méthacrylate de méthyle) : 80 g
- lubrifiant à base d'ester de colophane (cire E) : 10 g
- huile de soja époxydée : 40 g
- stéarate de calcium : 2,5 g
- stéarate de zinc : 2,5 g

Après homogénéisation en mélangeur rapide à froid, on prélève 6 fractions de cette composition B. A chaque fraction on ajoute une quantité de stéaroylbenzoylméthane (SBM) et éventuellement une quantité de diméthyl-2,6 bis(dodécyloxycarbonyl)-3,5 dihydro-1,4 pyridine (DHP art antérieur) ou de composé de l'invention préparé à l'exemple 1b (DHP ex 1b). Les quantités en poids pour 100 g de PVC sont indiquées dans le tableau II ci-après.

On prépare à l'aide des différentes compositions ainsi obtenues, ainsi qu'avec la composition B non modifiée, des feuilles de 1 mm d'épaisseur par malaxage sur un mélangeur à 2 cylindres pendant 3 min à 180°C.

A partir d'éprouvettes (environ 1 cm x 2 cm) découpées dans ces feuilles, on effectue un test de vieillissement thermique en étuve ventilée à 180°C et l'on suit en fonction du temps l'évolution de la coloration GARDNER.

Le tableau II rassemble les indices de coloration GARDNER mesurés pour différentes durées de vieillissement ainsi que la durée avant noircissement total des échantillons testés.

| ESSAIS | STABILISANTS | | INDICES GARDNER EN FONCTION DU TEMPS EN MIN | | | | | | | |
|---|---|---|---|---|---|---|---|---|---|---|
| | Nature | Poids en g/100g PVC | 0 | 7 | 14 | 21 | 30 | 45 | 60 | Noir a |
| Témoin | Néant | -- | 2 | 6 | 7 | 7 | 7 | 8 | 10 | 69 min |
| Exemple 5 | SBM<br>DHP ex 1b | 0,20<br>0,10 | 0 | 0 | 0 | 1 | 2 | 3 | 8 | 65 min |
| Essai B1 | SBM | 0,30 | 0 | 0 | 0,5 | 2 | 3,5 | 4 | 9 | 67 min |
| Essai B2 | SBM<br>DHP art antérieur | 0,20<br>0,10 | 0 | 0 | 0 | 1 | 2 | 3,5 | 9 | 64 min |
| Exemple 6 | SBM<br>DHP ex 1b | 0,20<br>0,20 | 0 | 0 | 0 | 1 | 1,5 | 3 | 9 | 64 min |
| Essai B3 | SBM | 0,40 | 0 | 0 | 0 | 1,5 | 3 | 4 | 9 | 66 min |
| Essai B3 | SBM<br>DHP art antérieur | 0,20<br>0,20 | 0 | 0 | 0 | 1 | 1,5 | 3,5 | 10 | 63 min |

TABLEAU II

Exemples 7 et 8 et essais comparatifs - Stabilisation UV du PVC

On prépare la composition de base B suivante :

- PVC LACQVYL SO 71 S : 1.000 g
- copolymère butadiène/styrène/méthacrylate de méthyle) : 80 g

- processing-aid (polymère acrylique de haute masse moléculaire) : 5 g
- huile de ricin hydrogène : 13 g
- lubrifiant cire E : 1,5 g
- lubrifiant à base de montanate de propylèneglycol partiellement saponifié (cire OP) : 4,0 g
- lubrifiant à base de cire de polyéthylène oxydé (cire AC 316) : 2,0 g
- stéarate de calcium : 2,3 g
- octanoate de zinc : 0,9 g
- huile de soja époxydée : 30 g

Après homogénéisation en mélangeur à froid, on prélève 6 fractions de cette composition C. A chaque fraction on ajoute différents stabilisants (indiqués dans le tableau III ci-après ainsi que les quantités en poids pour 100 g de PVC).

A l'aide de ces différentes compositions, on prépare des feuilles d'environ 1 mm d'épaisseur par malaxage sur un mélangeur à 2 cylindres pendant 3 min à 180°C.

A partir de ces feuilles, on prépare des films de 200 µm à l'aide d'une presse à plateaux à 185°C.

Ces films sont exposés dans une enceinte de vieillissement accéléré équipé d'un tube fluorescent émettant entre 290 nm et 400 nm, avec un maximum d'environ 360 nm.

L'enceinte est maintenue à 30°C et on mesure la coloration GARDNER sur les différents films après 19 h d'exposition.

Les résultats sont rassemblés dans le tableau III ci-après.

Les abréviations suivantes sont utilisées :

| | |
|---|---|
| SBM = | stéaroylbenzoylméthane ; |
| DHP art antérieur : | diméthyl-2,6 bis(dodécyloxycarbonyl)-3,5 dihydro-1,4 pyridine ; |
| DHP ex 1b : | composé de formule (I) préparé dans l'exemple 1b ; |
| Anti UV-E : | hydroxy-2 (hydroxy-2 éthoxy)-4 benzophénone (anti UV du type benzophénone très couramment utilisé). |

| Essais | Stabilisants (en g/100 g PVC) | | Coloration GARDNER pour 192 h d'exposition |
|---|---|---|---|
| Exemple 7 | DHP ex 1b | 0,25 | 2 |
| Essai C1 | DHP art antérieur | 0,25 | 6 |
| Essai C2 | SBM | 0,25 | 7 |
| Exemple 8 | SBM<br>DHP ex 1b | 0,125<br>0,25 | 2 |
| Essai C3 | SBM<br>DHP art antérieur | 0,125<br>0,25 | 6 |
| Essai C4 | SBM<br>Anti UV-E | 0,125<br>0,25 | 3 |

<u>TABLEAU III</u>

## Exemple 9 et essai comparatif

On prépare la composition de base D suivante :

- PVC LACQVYL SO 71 S : 1.000 g
- lubrifiant interne (mélange d'hexadécanol et d'octadécanol) : 14 g
- lubrifiant cire E : 2 g

- lubrifiant cire OP : 3 g
- stabilisant thiocétain * : 15 g

En opérant comme dans les exemples 7 et 8, on prépare des films de 200 µm contenant un stabilisant UV (comme indiqué dans le tableau IV ci-après) ou sans stabilisant UV (témoin).

Ces films sont placés dans une enceinte de vieillissement accéléré artificiel, à 58°C, équipée d'un tube fluorescent de type B 40 W émettant entre 275 nm et 380 nm, avec un maximum d'intensité à 310 nm.

On suit dans le spectre visible l'évolution de la densité optique des séquences polyéniques (contenant 10 doubles liaisons conjuguées) à une longueur d'onde λ = 447 nm.

Après 750 heures de vieillissement on obtient les valeurs indiquées dans le tableau (IV) ci-après.

| Essais | Stabilisants anti UV en g/100 g PVC | Densité optique a λ = 447 nm après 750 heures de vieillissement |
|---|---|---|
| Témoin | néant | 0,40 |
| Exemple 9 | DHP ex 1b | 0,15 |
| Essai D1 | Anti UVP * | 0,15 |

TABLEAU IV

* Anti UVP = hydroxy-2 octyloxy-4 benzophénone (stabilisant anti UV de type benzophénone très largement utilisé dans le PVC)

On remarque l'action anti UV très nette du composé de formule (I) préparé dans l'exemple 1b ; cette efficacité est équivalente à celle d'un anti UV de type benzophénone très largement utilisé. En outre cette efficacité existe dans une composition stabilisée thermiquement par un composé thioétain, dont l'action photosensibilisatrice est connue et importante.

## Revendications

### Revendications pour les Etats contractants suivants : AT, BE, CH, LI, DE, DK, FR, GB, GR, IT, LU, NL, SE

**1.** Composés à fonction dihydro-1,4 pyridine de formule générale (I) :

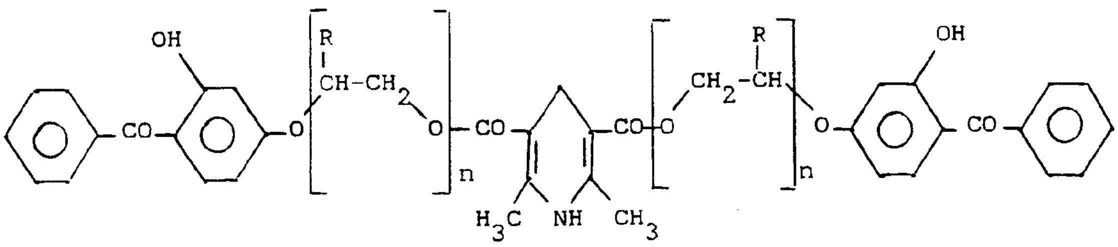

(I)

dans laquelle :

- n est un nombre de 1 à 3

(*mélange de 75 % en poids de dioctylstanniobis(sulfuroacétate d'isooctyle), et de 25 % en poids de trioctylstanniosulfuroacétate d'isooctyle)

- R représente un radical méthyle ou un atome d'hydrogène.

2. Composés selon la revendication 1, caractérisés en ce qu'ils sont choisis parmi :
   - la bis [(benzoyl-4 hydroxy-3 phénoxy)-2 éthoxycarbonyl]-3,5 diméthyl-2,6 dihydro-1,4 pyridine ;
   - la bis [(benzoyl-4 hydroxy-3 phénoxy)-2 méthyl-2 éthoxycarbonyl]-3,5 diméthyl-2,6 dihydro-1,4 pyridine.

3. Utilisation des composés selon l'une des revendications 1 ou 2 pour la stabilisation des polymères organiques.

4. Compositions à base de polymère chloré stabilisé, caractérisées en ce qu'elles contiennent :
   a) une quantité efficace d'au moins un composé organique du zinc,
   b) une quantité efficace d'au moins un composé organique du calcium, du baryum, du magnésium ou du strontium et/ou d'une hydrotalcite ;
   c) une quantité efficace d'au moins un composé à fonction dihydro-1,4 pyridine de formule (I) selon l'une des revendications 1 ou 2.

5. Compositions selon la revendication 4, caractérisées en ce qu'elles comprennent de 0,005 % à 5 % en poids de composé de formule (I) par rapport au polymère chloré, et de préférence de 0,01 % à 2 % en poids.

6. Compositions selon l'une des revendications 4 ou 5, caractérisées en ce qu'elles comprennent :
   - de 0,005 % à 1 % en poids d'au moins un composé organique du zinc par rapport au polymère chloré et de préférence de 0,01 % à 0,6 % en poids ;
   - de 0,005 % à 5 % en poids d'au moins un composé organique du calcium, du baryum, du magnésium et du strontium ou une hydrotalcite par rapport au polymère chloré et de préférence de 0,02 % à 2 % en poids.

7. Compositions selon l'une des revendications 4 à 6, caractérisées en ce qu'elles comprennent 0,005 % à 5 % en poids d'au moins une β-dicétone par rapport au polymère chloré et de préférence de 0,01 % à 2 % en poids.

8. Compositions selon l'une des revendications 4 à 7, caractérisées en ce qu'elles comprennent de 0,005 % à 1 % en poids de polyol par rapport au polymère chloré et de préférence de 0,01 % à 0,6 % en poids.

9. Compositions selon l'une des revendications 4 à 8, caractérisées en ce qu'elles comprennent de 0,05 % à 5 % en poids d'au moins un phosphite organique par rapport au polymère chloré et de préférence de 0,1 % à 2 % en poids.

10. Compositions à base de polymères organiques choisis parmi les polyoléfines, les polystyrènes, les polyalcadiènes, les polyuréthannes,
    les polyamides, les polyesters, les polycarbonates, les polysulfones, les polyéthers-sulfones, les polyéthers-cétones, les polymères acryliques, leurs copolymères et leurs mélanges, caractérisées en ce qu'elles comprennent de 0,005 % à 5 % en poids de composé de formule (I) selon l'une des revendications 1 ou 2 par rapport au poids de polymère organique et de préférence de 0,01 % à 2 % en poids.

11. Compositions à base de polymères organiques choisis parmi les polyoléfines et les polyalcadiènes tels que le polypropylène, le polyéthylène haute densité, le polyéthylène basse densité, le polyéthylène basse densité linéaire, le polybutadiène, leurs copolymères ou leurs mélanges, caractérisées en ce qu'elles comprennent de 0,005 % à 5 % en poids de composé de formule (I) selon l'une des revendications 1 ou 2 par rapport au poids de polymère organique et de préférence de 0,01 % à 2 % en poids.

**Revendications pour l'Etat contractant suivant : ES**

1. Procédé de préparation des composés à fonction dihydro-1,4 pyridine de formule générale (I) :

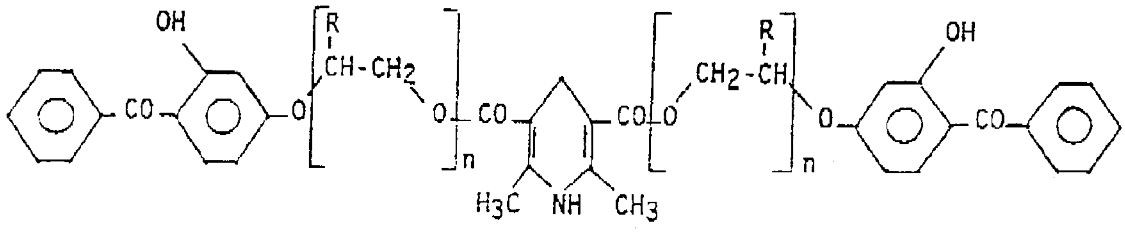

(I)

dans laquelle :
- n est un nombre de 1 à 3
- R représente un radical méthyle ou un atome d'hydrogène,

caractérisé en ce que l'on fait :
- réagir une hydroxy-2 hydroxyalcoxy-4 benzophénone ou une hydroxy-2 hydroxy (polyalcoxy)-4 benzophénone et le dicétène pour former l'acétoacétate correspondant de la benzophénone substituée,
- puis réagir l'acétoacétate ainsi obtenu avec de l'acétate d'ammonium en présence d'hexaméthylène tétramine.

2. Procédé de préparation des composés à fonction dihydro-1,4 pyridine de formule générale (I) :

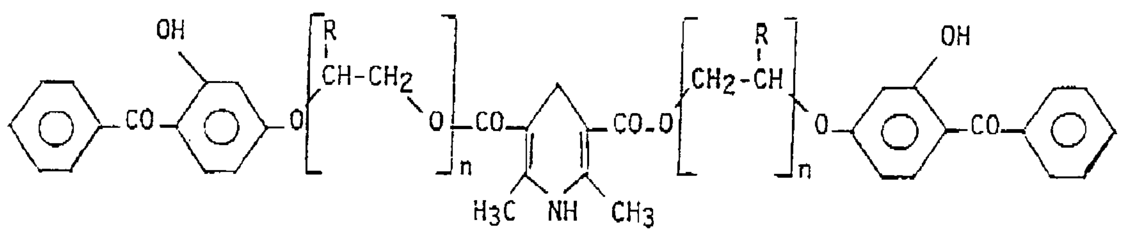

(I)

dans laquelle :
- n est un nombre de 1 à 3
- R représente un radical méthyle ou un atome d'hydrogène, caractérisé en ce qu'il comprend les étapes suivantes :
- réaction d'une hydroxy-2 hydroxyalcoxy-4 benzophénone ou d'une hydroxy-2 hydroxy (polyalcoxy)-4 benzophénone et du dicétène pour former l'acétoacétate correspondant de la benzophénone substituée,
- réaction du formaldéhyde sur l'acétoacétate conduisant au méthylène bis-acétoacétate de la benzophénone substituée ;
- cyclisation de ce dernier composé en dihydro-1,4 pyridine correspondante au moyen d'ammoniac.

3. Procédé selon la revendication 2, caractérisé en ce que l'on inverse les deux dernières étapes en faisant réagir le formaldéhyde sur le β-aminocrotonate dérivant de l'acétoacétate et de l'ammoniac.

4. Procédé selon l'une des revendications 1 à 3, caractérisé en ce qu'il est appliqué à la préparation de :
- la bis [(benzoyl-4 hydroxy-3 phénoxy)-2 éthoxycarbonyl]-3,5 diméthyl-2,6 dihydro-1,4 pyridine ;
- la bis [(benzoyl-4 hydroxy-3 phénoxy)-2 méthyl-2 éthoxycarbonyl]-3,5 diméthyl-2,6 dihydro-1,4 pyridine.

5. Utilisation des composés préparés selon l'une des revendications 1 à 4 pour la stabilisation des polymères organiques.

6. Compositions à base de polymère chloré stabilisé, caractérisées en ce qu'elles contiennent :

a) une quantité efficace d'au moins un composé organique du zinc,

b) une quantité efficace d'au moins un composé organique du calcium, du baryum, du magnésium ou du strontium et/ou d'une hydrotalcite ;

c) une quantité efficace d'au moins un composé à fonction dihydro-1,4 pyridine de formule (I) préparé selon l'une des revendications 1 à 4.

7. Compositions selon la revendication 6, caractérisées en ce qu'elles comprennent de 0,005 % à 5 % en poids de composé de formule (I) par rapport au polymère chloré, et de préférence de 0,01 % à 2 % en poids.

8. Compositions selon l'une des revendications 6 ou 7, caractérisées en ce qu'elles comprennent :
   - de 0,005 à 1 % en poids d'au moins un composé organique du zinc par rapport au polymère chloré et de préférence de 0,01 % à 0,6 % en poids ;
   - de 0,005 % à 5 % en poids d'au moins un composé organique du calcium, du baryum, du magnésium et du strontium ou une hydrotalcite par rapport au polymère chloré et de préférence de 0,02 % à 2 % en poids.

9. Compositions selon l'une des revendications 6 à 8, caractérisées en ce qu'elles comprennent 0,005 % à 5 % en poids d'au moins une β-dicétone par rapport au polymère chloré et de préférence de 0,01 % à 2 % en poids.

10. Compositions selon l'une des revendications 6 à 9, caractérisées en ce qu'elles comprennent de 0,005 % à 1 % en poids de polyol par rapport au polymère chloré et de préférence de 0,01 % à 0,6 % en poids.

11. Compositions selon l'une des revendications 6 à 10, caractérisées en ce qu'elles comprennent de 0,05 % à 5 % en poids d'au moins un phosphite organique par rapport au polymère chloré et de préférence de 0,1 % à 2 % en poids.

12. Compositions à base de polymères organiques choisis parmi les polyoléfines, les polystyrènes, les polyalcadiènes, les polyuréthannes, les polyamides, les polyesters, les polycarbonates, les polysulfones, les polyéthers-sulfones, les polyéthers-cétones, les polymères acryliques, leurs copolymères et leurs mélanges, caractérisées en ce qu'elles comprennent de 0,005 % à 5 % en poids de composé de formule (I) préparé selon l'une des revendications 1 à 4 par rapport au poids de polymère organique et de préférence de 0,01 % à 2 % en poids.

13. Compositions à base de polymères organiques choisis parmi les polyoléfines et les polyalcadiènes tels que le polypropylène, le polyéthylène haute densité, le polyéthylène basse densité, le polyéthylène basse densité linéaire, le polybutadiène, leurs copolymères ou leurs mélanges, caractérisées en ce qu'elles comprennent de 0,005 % à 5 % en poids de composé de formule (I) préparé selon l'une des revendications 1 à 4 par rapport au poids de polymère organique et de préférence de 0,01 % à 2 % en poids.

## Claims

### Claims for the following Contracting States : AT, BE, CH, LI, DE, DK, FR, GB, GR, IT, LU, NL, SE

1. Compounds having a 1,4-dihydropyridine function of the general formula (I):

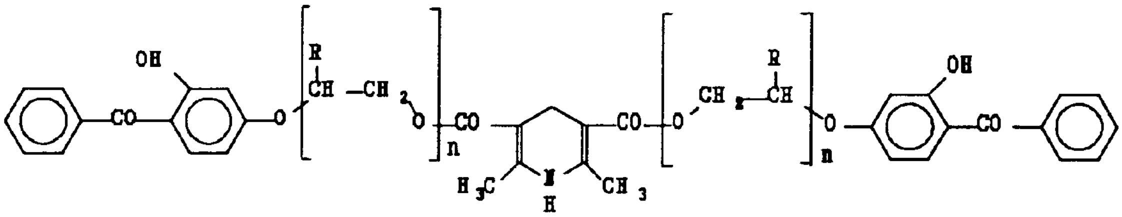

(I)

in which:
   - n is a number ranging from 1 to 3,
   - R represents a methyl radical or a hydrogen atom.

2. Compounds according to claim 1, characterized by the fact that they are selected among:
   - 3,5-bis[2-(4-benzoyl-3-hydroxyphenoxy)ethoxycarbonyl]-2,6-dimethyl-1,4-dihydropyridine;
   - 3,5-bis[2-(4-benzoyl-3-hydroxyphenoxy) -2-methylethoxycarbonyl]-2,6-dimethyl-1,4-dihydropyridine.

3. Use of compounds according to one of claims 1 or 2 for the stabilizing of organic polymers.

4. Compositions based on a stabilized chlorinated polymer, characterized by the fact that they comprise:
   a) an effective amount of at least one organic zinc compound,
   b) an effective amount of at least one organic calcium, barium, magnesium or strontium compound and/or of a hydrotalcite;
   c) an effective amount of at least one compound having a 1,4-dihydropyridine function of formula (I) according to one of claims 1 or 2.

5. Compositions according to claim 4, characterized by the fact that they comprise from 0.005% to 5% by weight of a compound of formula (I) relative to the chlorinated polymer, and preferably from 0.01% to 2% by weight.

6. Compositions according to one of claims 4 or 5, characterized by the fact that they comprise:
   - from 0.005% to 1% by weight of at least one organic zinc compound relative to the chlorinated polymer, and preferably from 0.01% to 0.6% by weight;
   - from 0.005% to 5% by weight of at least one organic calcium, barium, magnesium or strontium compound or of a hydrotalcite relative to the chlorinated polymer, and preferably from 0.02% to 2% by weight.

7. Compositions according to one of claims 4 to 6, characterized by the fact that they comprise from 0.005% to 5% by weight of at least one β-diketone relative to the chlorinated polymer, and preferably from 0.01% to 2% by weight.

8. Compositions according to one of claims 4 to 7, characterized by the fact that they comprise from 0.005% to 1% by weight of polyol relative to the chlorinated polymer, and preferably from 0.01% to 0.6% by weight.

9. Compositions according to one of claims 4 to 8, characterized by the fact that they comprise from 0.05% to 5% by weight of at least one organic phosphite relative to the chlorinated polymer, and preferably from 0.1% to 2% by weight.

10. Compositions based on organic polymers selected from polyolefins, polystyrenes, polyalkadienes, polyurethanes, polyamides, polyesters, polycarbonates, polysulfones, polyethersulfones, polyetherketones, acrylic polymers, the copolymers and mixtures thereof, characterized by the fact that they comprise from 0.005% to 5% by weight of compound of formula (I) according to one of claims 1 or 2 relative to the weight of the organic polymer, and preferably from 0.01% to 2% by weight.

11. Compositions based on organic polymers selected from polyolefins and polyalkadienes such as polypropylene, high density polyethylene, low density polyethylene, linear low density polyethylene, polybutadiene, the copolymers or mixtures thereof, characterized by the fact that they comprise from 0.005% to 5% by weight of compound of formula (I) according to one of claims 1 or 2 relative to the weight of the organic polymer, and preferably from 0.01% to 2% by weight.

**Claims for the following Contracting State : ES**

1. Process for the preparation of compounds, having a 1,4-dihydropyridine function, of the general formula (I):

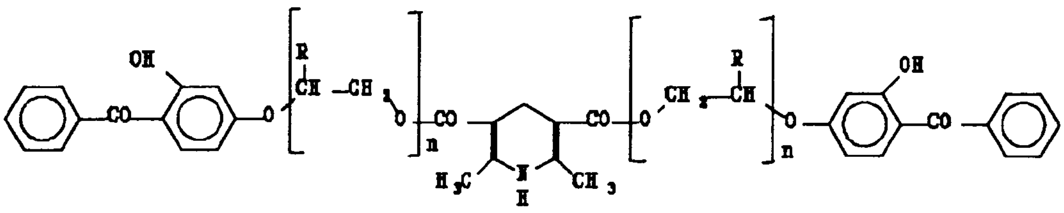

(I)

in which:

- n is a number ranging from 1 to 3,
- R represents a methyl radical or a hydrogen atom,

characterized in that

- 2-hydroxy-4-hydroxyalkoxybenzophenone or 2-hydroxy-4-hydroxy(polyalkoxy)benzophenone and diketene are reacted to form the corresponding acetoacetate of the substituted benzophenone,
- the acetoacetate thus obtained is then reacted with ammonium acetate in the presence of hexamethylenetetramine.

**2.** Process for the preparation of compounds, having a 1,4-dihydropyridine function, of the general formula (I):

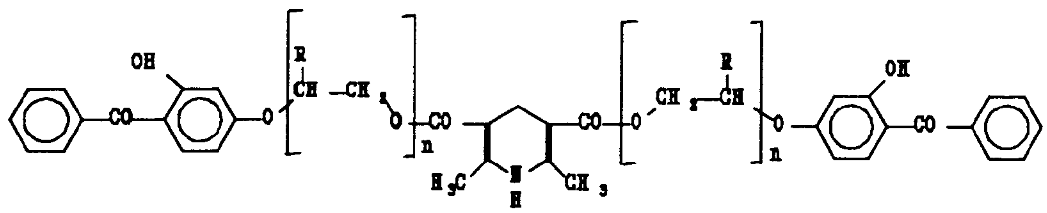

(I)

in which:

- n is a number ranging from 1 to 3,
- R represents a methyl radical or a hydrogen atom,

characterized in that it comprises the following stages:

- reaction of 2-hydroxy-4-hydroxyalkoxybenzophenone or 2-hydroxy-4-hydroxy(polyalkoxy)benzophenone and diketene to form the corresponding acetoacetate of the substituted benzophenone,
- reaction of formaldehyde with the acetoacetate, producing the methylene-bis-acetoacetate of the substituted benzophenone;
- ring closure of this latter compound to the corresponding 1,4-dihydropyridine, using ammonia.

**3.** Process according to claim 2, characterized in that the two last stages are reversed to react formaldehyde with the β-aminocrotonate derived from the acetoacetate and ammonia.

**4.** Process according to one of claims 1 to 3, characterized in that it is applied to the preparation of

- 3,5-bis[2-(4-benzoyl-3-hydroxyphenoxy)ethoxycarbonyl]-2,6-dimethyl-1,4-dihydropyridine;
- 3,5-bis[2-(4-benzoyl-3-hydroxyphenoxy)-2-methylethoxycarbonyl]-2,6-dimethyl-1,4-dihydropyridine.

**5.** Use of compounds according to one of claims 1 to 4 for the stabilizing of organic polymers.

**6.** Compositions based on a stabilized chlorinated polymer, characterized in that they comprise:

a) an effective amount of at least one organic zinc compound,

b) an effective amount of at least one organic calcium, barium, magnesium or strontium compound and/or of a hydrotalcite;

c) an effective amount of at least one compound having a 1,4-dihydropyridine function of formula (I) according to one of claims 1 to 4.

**7.** Compositions according to claim 6, characterized in that they comprise from 0.005% to 5% by weight of a compound of formula (I) relative to the chlorinated polymer, and preferably from 0.01% to 2% by weight.

8. Compositions according to one of claims 6 or 7, characterized in that they comprise:
   - from 0.005% to 1% by weight of at least one organic zinc compound relative to the chlorinated polymer, and preferably from 0.01% to 0.6% by weight;
   - from 0.005% to 5% by weight of at least one organic calcium, barium, magnesium or strontium compound or of a hydrotalcite relative to the chlorinated polymer, and preferably from 0.02% to 2% by weight.

9. Compositions according to one of claims 6 to 8, characterized in that they comprise from 0.005% to 5% by weight of at least one β-diketone relative to the chlorinated polymer, and preferably from 0.01% to 2% by weight.

10. Compositions according to one of claims 6 to 9, characterized in that they comprise from 0.005% to 1% by weight of polyol relative to the chlorinated polymer, and preferably from 0.01% to 0.6% by weight.

11. Compositions according to one of claims 6 to 10, characterized in that they comprise from 0.05% to 5% by weight of at least one organic phosphite relative to the chlorinated polymer, and preferably from 0.1% to 2% by weight.

12. Compositions based on organic polymers selected from polyolefins, polystyrenes, polyalkadienes, polyurethanes, polyamides, polyesters, polycarbonates, polysulfones, polyethersulfones, polyetherketones, acrylic polymers, the copolymers and mixtures thereof, characterized in that they comprise from 0.005% to 5% by weight of compound of formula (I) prepared according to one of claims 1 to 4 relative to the weight of the organic polymer, and preferably from 0.01% to 2% by weight.

13. Compositions based on organic polymers selected from polyolefins and polyalkadienes such as polypropylene, high density polyethylene, low density polyethylene, linear low density polyethylene, polybutadiene, the copolymers or mixtures thereof, characterized in that they comprise from 0.005% to 5% by weight of compound of formula (I) prepared according to one of claims 1 to 4 relative to the weight of the organic polymer, and preferably from 0.01% to 2% by weight.

## Patentansprüche

### Patentansprüche für folgende Vertragsstaaten : AT, BE, CH, LI, DE, DK, FR, GB, GR, IT, LU, NL, SE

1. Verbindungen, die eine Dihydro-1,4-pyridin-Funktion der allgemeinen Formel (I) enthalten:

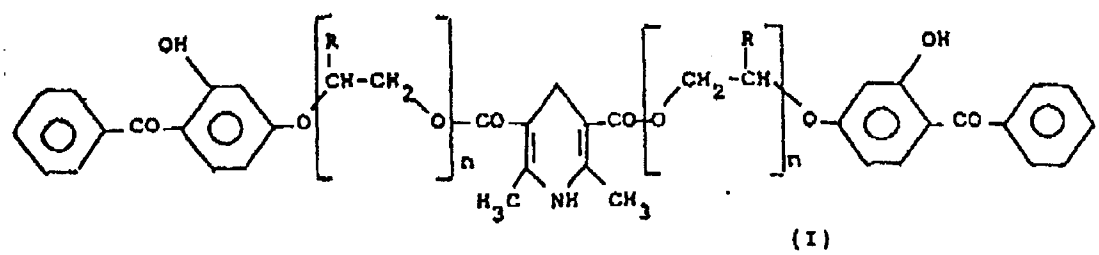

(I)

worin n eine Zahl von 1 bis 3 ist,
R einen Methylrest oder ein Wasserstoffatom darstellt.

2. Verbindungen gemäß Anspruch 1,
**dadurch gekennzeichnet,** daß sie ausgewählt sind aus:
dem Bis-[(benzoyl-4-hydroxy-3-phenoxy)-2-ethoxycarbonyl]-3,5-dimethyl-2,6-dihydro-1,4-pyridin;
dem Bis-[(benzoyl-4-hydroxy-3-phenoxy)-2-methyl-2-ethoxycarbonyl)-3,5-dimethyl-2,6-dihydro-1,4-pyridin.

3. Verwendung von Verbindungen gemäß einem der Ansprüche 1 oder 2 zur Stabilisierung von organischen Polymeren.

4. Zusammensetzungen auf Basis von stabiliertem chlorhaltigem Polymer,
**dadurch gekennzeichnet**, daß sie enthalten:
(a) eine wirksame Menge mindestens einer zinkorganischen Verbindung,
(b) eine wirksame Menge mindestens einer organischen Verbindung von Calcium, Barium, Nagnesium oder Strontium und/oder Hydrotalcits;
(c) eine wirksame Menge mindestens einer Verbindung mit einer Dihydro-1,4-pyridin-Funktion der allgemeinen Formel (I) gemäß einem der Ansprüche 1 oder 2.

5. Zusammensetzungen gemäß Anspruch 4,
**dadurch gekennzeichnet**, daß sie 0,005 bis 5 Gew.-% der Verbindung der Formel (I), bezogen auf das chlorhaltige Polymer, und vorzugsweise 0,01 bis 2 Gew.-% umfassen.

6. Zusammensetzungen gemäß einem der Ansprüche 4 oder 5,
**dadurch gekennzeichnet,** daß sie enthalten:
- 0,005 bis 1 Gew.-% mindestens einer zinkorganischen Verbindung, bezogen auf das chlorhaltige Polymer und vorzugsweise 0,01 bis 0,6 Gew.-%;
- 0,005 bis 5 Gew.-% mindestens einer organischen Verbindung von Calcium, Barium, Magnesium oder Strontium oder ein Hydrotalcit, bezogen auf das chlorhaltige Polymer, und vorzugsweise 0,02 bis 2 Gew.-%.

7. Zusammensetzungen gemäß einem der Ansprüche 4 bis 6,
**dadurch gekennzeichnet,** daß sie 0,005 bis 5 Gew.-% mindestens eines β-Diketone, bezogen auf das chlorhaltige Polymer, und vorzugsweise 0,01 bis 2 Gew.-% umfassen.

8. Zusammensetzungen gemäß einem der Ansprüche 4 bis 7,
**dadurch gekennzeichnet** daß sie 0,005 bis 1 Gew.-% Polyol, bezogen auf das chlorhaltige Polymer, und vorzugsweise 0,01 bis 0,6 Gew.-% umfassen.

9. Zusammensetzungen gemäß einem der Ansprüche 4 bis 8,
**dadurch gekennzeichnet** daß sie 0,05 bis 5 Gew.-% mindestens eines organischen Phosphits, bezogen auf das chlorhaltige Polymer, und vorzugsweise 0,1 bis 2 Gew.-% umfassen.

10. Zusammensetzungen auf Basis von organischen Polymeren, die ausgewählt sind aus den Polyolefinen, den Polystyrolen, den Polyalkadienen, den Polyurethanen, den Polyamiden, den Polyestern, den Polycarbonaten, den Polysulfanen, den Polyethersulfonen, den Polyetherketonen, den Acrylpolymeren, deren Copolymeren und deren Mischungen,
**dadurch gekennzeichnet** daß sie 0,005 bis 5 Gew.-% der Verbindung der Formel (I), gemäß einem der Ansprüche 1 oder 2, bezogen auf das Gewicht des organischen Polymers, und vorzugsweise 0,01 bis 2 Gew.-% umfassen.

11. Zusammensetzungen auf Basis von organischen Polymeren, ausgewählt aus den Polyolefinen und den Polyalkadienen, wie dem Polypropylen, dem Polyethylen hoher Dichte, dem Polyethylen niedriger Dichte, dem linearen Polyethylen niedriger Dichte, dem Polybutadien, deren Copolymeren oder deren Mischungen,
**dadurch gekennzeichnet**, daß sie 0,005 bis 5 Gew.-% der Verbindung der Formel (I) gemäß einem der Ansprüche 1 oder 2, bezogen auf das Gewicht des organischen Polymers und vorzugsweise 0,01 bis 2 Gew.-% umfassen.

**Patentansprüche für folgenden Vertragsstaat : ES**

1. Verfahren zur Herstellung von Verbindungen enthaltend eine 1,4-Dihydropyridin-Funktion und entsprechend der allgemeinen Formel (I):

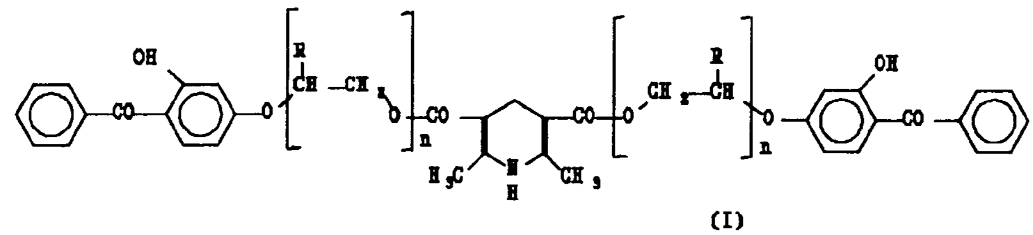

(I)

worin:

- n eine Zahl von 1 bis 3 ist,
- R einen Methylrest oder ein Wasserstoffatom darstellt,

**dadurch gekennzeichnet,** daß man

- ein 2-Hydroxy-4-hydroxyalkoxybenzophenon oder ein 2-Hydroxy-4-hydroxy(polyalkoxy)benzophenon und das Diketen reagieren läßt, zur Bildung des ensprechenden Acetoacetats des substituierten Benzophenons,
- das so erhaltene Acetoacetat mit Ammoniumacetat in Gegenwart von Hexamethylentetramin reagieren läßt.

**2.** Verfahren zur Herstellung von Verbindungen enthaltend eine 1,4-Dihydropyridin-Funktion und entsprechend der allgemeinen Formel (I):

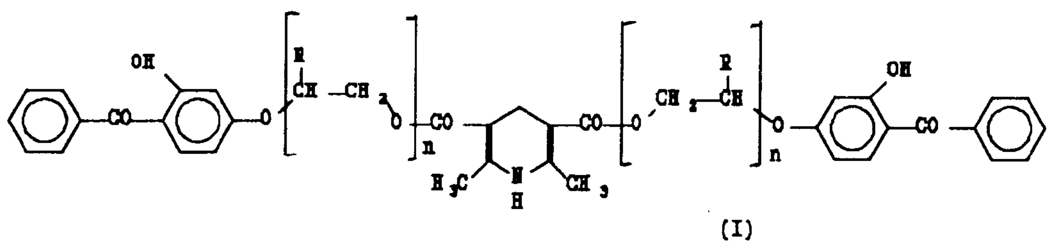

(I)

worin:

- n eine Zahl von 1 bis 3 ist,
- R einen Methylrest oder ein Wasserstoffatom darstellt,

**dadurch gekennzeichnet**, daß es die folgenden Stufen umfasst:

- Reaktion zwischen einem 2-Hydroxy-4-hydroxyalkoxybenzophenon oder einem 2-Hydroxy-4-hydroxy(polyalkoxy)benzophenon und Diketen, zur Bildung des ensprechenden Acetoacetats des substituierten Benzophenons,
- Reaktion von Formaldehyd mit Acetoacetat, zum Methylen-bis-acetoacetat des substituierten Benzophenon;
- Cyclisierung dieser letztgenannten Verbindung zum entsprechenden 1,4-Dihydropyridin mittels Ammoniak.

**3.** Verfahren gemäß Anspruch 2, **dadurch gekennzeichnet**, daß man die beiden letzten Stufen in der umgekehrten Reihenfolge durchführt, wobei man das Formaldehyd mit dem aus Acetoacetat und Ammoniak herrührenden β-Aminocrotonat reagieren läßt.

**4.** Verfahren gemäß einem der Ansprüche 1 bis 3, **dadurch gekennzeichnet,** daß es zur Herstellung

- des Bis-3,5-[2-(4-benzoyl-3-hydroxyphenoxy)-ethoxycarbonyl]-2,6-dimethyl-1,4-dihydropyridins;
- des Bis-3,5-[2-(4-benzoyl-3-hydroxyphenoxy)-2-methylethoxycarbonyl]-2,6-dimethyl-1,4-dihydropyridins

verwendet wird.

**5.** Verwendung von Verbindungen gemäß einem der Ansprüche 1 bis 4 zur Stabilisierung von organischen Polymeren.

**6.** Zusammensetzungen auf Basis von stabilisiertem chlorhaltigem Polymer, **dadurch gekennzeichnet**, daß sie enthalten:

a) eine wirksame Menge mindestens einer zinkorganischen Verbindung,
b) eine wirksame Menge mindestens einer organischen Verbindung von Calcium, Barium, Magnesium oder Strontium und/oder Hydrotalcits;
c) eine wirksame Menge mindestens einer Verbindung mit einer Dihydro-1,4-pyridin-Funktion der allgemeinen Formel (I) hergestellt gemäß einem der Ansprüche 1 bis 4.

**7.** Zusammensetzungen gemäß Anspruch 6, **dadurch gekennzeichnet**, daß sie 0,005 bis 5 Gew.-% der Verbindung der Formel (I), bezogen auf das chlorhaltige Polymer, und vorzugsweise 0,01 bis 2 Gew.-% umfassen.

**8.** Zusammensetzungen gemäß einem der Ansprüche 6 oder 7, **dadurch gekennzeichnet,** daß sie enthalten:
- 0,005 bis 1 Gew.-% mindestens einer zinkorganischen Verbindung, bezogen auf das chlorhaltige Polymer und vorzugsweise 0,01 bis 0,6 Gew.-%;
- 0,005% bis 5 Gew.-% mindestens einer organischen Verbindung von Calcium, Barium, Magnesium oder Strontium oder ein Hydrotalcit, bezogen auf das chlorhaltige Polymer, und vorzugsweise 0,02 bis 2 Gew.-%.

**9.** Zusammensetzungen gemäß einem der Ansprüche 6 bis 8, **dadurch gekennzeichnet**, daß sie 0,005% bis 5 Gew.-% mindestens eines β-Diketons, bezogen auf das chlorhaltige Polymer, und vorzugsweise 0,01 bis 2 Gew.-% umfassen.

**10.** Zusammensetzungen gemäß einem der Ansprüche 6 bis 9, **dadurch gekennzeichnet**, daß sie 0,005% bis 1 Gew.-% Polyol, bezogen auf das chlorhaltige Polymer, und vorzugsweise 0,01 bis 0,6 Gew.-% umfassen.

**11.** Zusammensetzungen gemäß einem der Ansprüche 6 bis 10, **dadurch gekennzeichnet**, daß sie 0,05% bis 5 Gew.-% mindestens eines organischen Phosphits, bezogen auf das chlorhaltige Polymer, und vorzugsweise 0,1 bis 2 Gew.-% umfassen.

**12.** Zusammensetzungen auf Basis von organischen Polymeren, die ausgewählt sind aus den Polyolefinen, den Polystyrolen, den Polyalkadienen, den Polyurethanen, den Polyamiden, den Polyestern, den Polycarbonaten, den Polysulfonen, den Polyethersulfonen, den Polyetherketonen, den Acrylpolymeren, deren Copolymeren und deren Mischungen, **dadurch gekennzeichnet**, daß sie 0,005 bis 5 Gew.-% der Verbindung der Formel (I), hergestellt gemäß einem der Ansprüche 1 bis 4, bezogen auf das Gewicht der organischen Polymers, und vorzugsweise 0,01 bis 2 Gew.-% umfassen.

**13.** Zusammensetzungen auf Basis von organischen Polymeren, ausgewählt aus den Polyolefinen und den Polyalkadienen wie dem Polypropylen, dem Polyethylen hoher Dichte, dem Polyethylen niedriger Dichte, dem linearen Polyethylen niedriger Dichte, dem Polybutadien, deren Copolymeren oder deren Mischungen, **dadurch gekennzeichnet**, daß sie 0,005 bis 5 Gew.-% der Verbindung der Formel (I) hergestellt gemäß einem der Ansprüche 1 bis 4, bezogen auf das Gewicht des organischen Polymers, und vorzugsweise 0,01 bis 2 Gew.-% umfassen.